# EUROPEAN PATENT APPLICATION

(11) **EP 1 146 051 A2**
(43) Date of publication of application: **17.10.2001**
(21) Application number: 01303172.9
(22) Date of filing: 03.04.2001
(51) Int. Cl.: C07H 17/08, A61K 31/70, A61P 31/04, A61P 33/02

(54) **Erythromycin A derivatives**

(30) Priority: 10.04.2000 US 196045 P
(71) Applicant: Pfizer Products Inc., Groton, Connecticut 06340 (US)
(72) Inventor: Kaneko, Takushi, c/o Pfizer Global Res and Dev, Groton, Connecticut 06340 (US); McMillen, William Thomas, Indiana 46032 (US); Wu, Yong-Jin, Connecticut 06443 (US)
(74) Representative: Motion, Keith Robert

(57) **Abstract**

The present invention relates to compounds of the formula 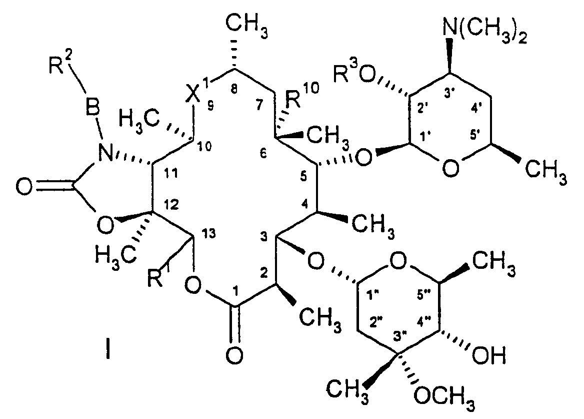 and to pharmaceutically acceptable salts, prodrugs, tautomers, and solvates, thereof, wherein R¹, R², R³, R¹⁰, B and X¹ are defined as described herein.

## Description

### Background of the Invention

This invention relates to novel macrolide compounds that are useful as antibacterial and antiprotozoal agents in mammals, including man, as well as in fish and birds. This invention also relates to methods of preparing the novel compounds, novel intermediates useful in preparation of the compounds, and pharmaceutical compositions containing the novel compounds. In addition, the present invention includes methods of treating bacterial and protozoal infections through the administration of the novel compounds to mammals, fish and birds requiring such treatment.

Derivatives of erythromycin A that are useful as antibiotic agents are referred to in U.S. patent application serial number WO 98/56800, published December 17, 1998; WO 98/51696, published November 19, 1998; WO 99/21866, published May 6, 1999; WO 99/62920, published December 9, 1999; EP 895999, published February 10, 1999; WO 99/21865, published May 6, 1999; U.S. patent application serial number 60/117,342, filed January 27, 1999; U.S. patent application serial number 60/130,809, filed April 23, 1999; U.S. patent application serial number 60/130,912, filed April 23, 1999; and U.S. patent application serial number 60/130,913, filed April 23, 1999. Derivatives of erythromycin A are also referred to in United States patents 4,474,768 and 4,517,359, relating to the commercially available antibiotic azithromycin. These patents and patent applications are incorporated herein by reference in their entireties.

### Summary of the Invention

The present invention relates to compounds of the formula 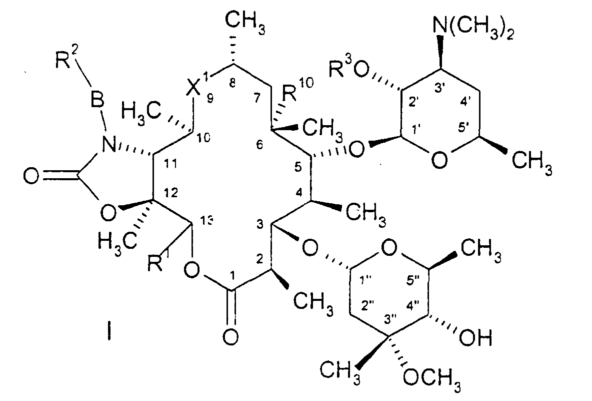 and to pharmaceutically acceptable salts, prodrugs, tautomers, and solvates, thereof, wherein:
X¹ is selected from -C(O)-, -CH(NR⁵R⁶)-, -CHR⁵NR⁶-, -NR⁶CHR⁵-, -C(=NR⁵)- and-C(=N-OR⁵)-, wherein the first dash of each of the foregoing X¹ groups is attached to the C-10 carbon of the compound of formula I and the last dash of each group is attached to the C-8 carbon of the compound of formula I;
B is selected from O, (CR^{aa}R^{bb})ₘ, SO₂, and NR⁴, wherein m is 0 or 1;
R^{aa} and R^{bb} are independently selected from H and C₁-C₆ alkyl;
R^{aa} and R^{bb} together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-, -N(C₁-C₆) alkyl-, and -C(O)- and are optionally substituted by one or more substituents selected from the group T substituents;
when B is NR⁴, B and R² together with the nitrogen to which they are attached can form a 3- to 10-membered ring wherein one or more carbons of said ring are optionally replaced by one or more diradicals selected from -O-, -S-, -S(O)-, -S(O)₂-, -NH-, -N(C₁-C₆) alkyl- and -C(O)- and are optionally substituted by one or more substituents selected from the group T substituents;
when B is NR⁴, B and R² together with the nitrogen to which they are attached can form -N=C(R⁴)(CR^{a}R^{b})ₙAr, wherein n is an integer ranging from 0 to 10;
B, R² and X¹ can be taken together;
when taken together, B, R² and X¹ taken with their intervening atoms form an additional two rings having one of the following structures: 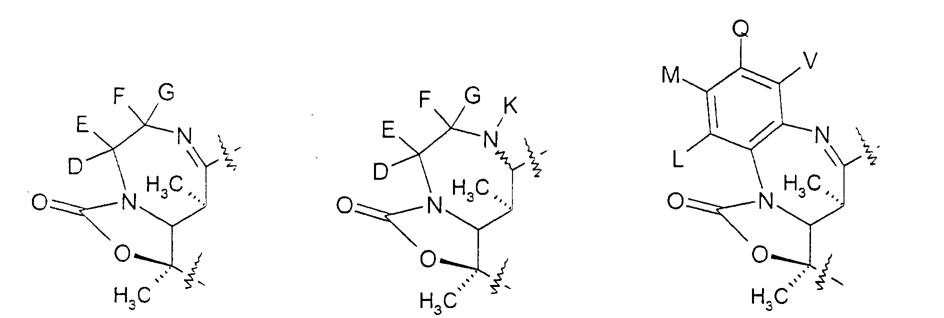 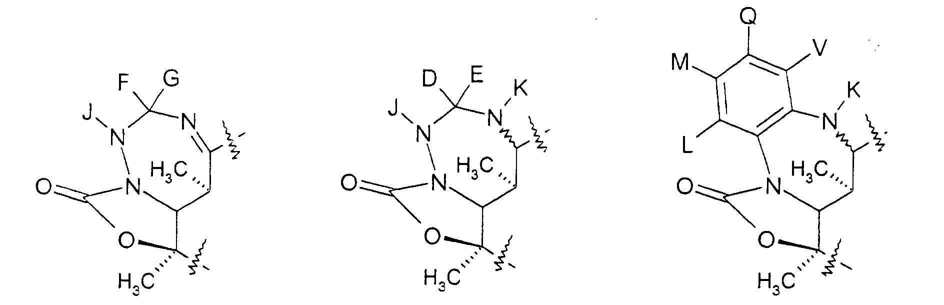 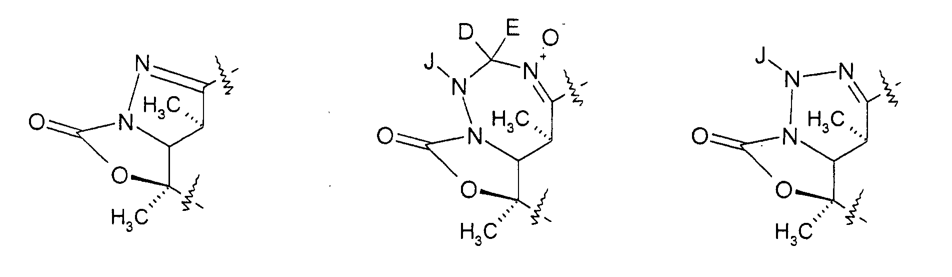 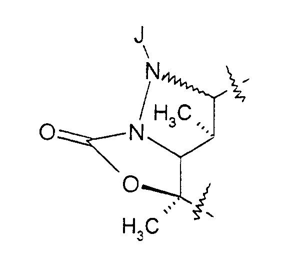 wherein:
   each of D, E, F and G is independently selected from H, halo, C₁-C₁₂ alkyl, C₃-C₁₀ alkenyl, C₃-C₁₀ alkynyl and (CR^{a}R^{b})ₙAr, wherein n is an integer ranging from 0 to 10, wherein one or more carbons of said alkyl are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
   D and E or F and G together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
   each of J, J¹ and K is independently selected from C(O)R⁵, C(O)NR⁵R⁶, C(O)OR⁵, (CR^{a}R^{b})ₙAr, O(CR^{a}R^{b})ₙAr and N(CR^{a}R^{b})ₙAr; wherein n is an integer ranging from 0 to 10;
   each of L, M, Q and V is independently selected from H and the group T substituents;
   one or two carbons of the phenyl ring in which L, M, Q and V are attached can be replaced with nitrogen and L, M, Q, and V do not indicate any substituent where attached to nitrogen;
   R¹ is C₁-C₂₀ alkyl, C₆-C₁₀ aryl, or C₇-C₁₄ aralkyl, wherein one or more carbons of said alkyl are optionally replaced by one or more diradicals selected from -O-, -S-, -S(O)-, -S(O)₂-, -NH-, -N(C₁₋C₆)alkyl- and -C(O)- and are optionally substituted by one or more substituents selected from the group T substituents;
   Ar is independently a 3- to 10-membered heterocyclic or C₅-C₁₀ aryl, wherein said heterocyclic and aryl groups are optionally substituted by one or more substituents independently selected from the group T substituents;
   T substituents are selected from the group consisting of:
      (a) nitro;
      (b) halo;
      (c) hydroxy:
      (d) N₃;
      (e) CN;
      (f) CHO;
      (g) C₁-C₁₀alkoxy;
      (h) C₁-C₃ alkoxy-C₁-C₃ alkoxy;
      (i) oxo;
      (j) C₁-C₁₀ alkanoyl;
      (k) C₁-C₁₀ alkyl;
      (I) C₁-C₁₂ alkyl substituted with an aromatic 5- to 10-membered heterocyclic;
      (m) C₁-C₆ alkyl substituted with O-SO₂;
      (n) C₂-C₁₀ alkenyl;
      (o) C₂-C₁₀ alkynyl;
      (p) C₃-C₁₀ cycloalkyl;
      (q) substituted* C₃-C₁₀ cycloalkyl;
      (r) 3- to 10-membered heterocyclic;
      (s) substituted* 3- to 10-membered heterocyclic;
      (t) C₅-C₁₀ aryl;
      (u) substituted* C₅-C₁₀ aryl;
      (v) tri C₁-C₆ alkylsilyl;
      (w) -C(O)R⁵:
      (x) -C(O)₂R⁷;
      (y) -C(O)OR⁵;
      (z) -OC(O)R⁵
      (aa) -C(O)NR⁵R⁶;
      (bb) -OC(O)NR⁵R⁶;
      (cc) -O(C)OR⁵;
      (dd) -NR⁵R⁶;
      (ee) -NR⁸R⁹;
      (ff) -NHC(O)R⁵;
      (gg) -NHC(O)NR⁵R⁶;
      (hh) =N-O-R⁵;
      (ii) =N-NR⁵R⁶;
      (jj) =N-NR⁸R⁹;
      (kk) =N-R⁵;
      (II) =N-R⁷;
      (mm) =N-NHC(O)R⁵;
      (nn) =N-NHC(O)NR⁵R⁶;
      (oo) -C≡N;
      (pp) -S(O)ₙ, wherein n is 0, 1 or 2;
      (qq) -S(O)ₙR⁵, wherein n is 0, 1 or 2;
      (rr) -O-S(O)ₙR⁵, wherein n is 0, 1 or 2; and
      (ss) -SO₂NR⁵R⁶;

Substituted groups in this list can be substituted with T substituents (i.e., with T substituents other than those indicated as "substituted").
R² is selected from H, C₁-C₁₂ alkyl, C₃-C₁₀ alkenyl, C₃-C₁₀ alkynyl and (CR^{a}R^{b})ₙAr wherein n is an integer ranging from 0 to 10, and wherein one or more carbons of said alkyl are optionally replaced by one or more diradicals selected from -O-, -S-, -S(O)-, -S(O)₂-, -NH-, -N(C₁-C₆)alkyl-, and -C(O)- and are optionally substituted by one or more substituents selected from the group T substituents;
R³ is selected from H, C(O)(C₁-C₁₈)alkyl, C(O)Ar, a OC(O)(C₁-C₁₈)alkyl and OC(O)Ar, wherein the alkyl moieties of the foregoing R³ groups are optionally replaced by one or more diradicals selected from -O-, -S-, -S(O)-. -S(O)₂-, -NH-, -N(C₁-C₆)alkyl- and -C(O)- and are optionally substituted by one or more substituents selected from the group T substituents;
each of R⁴, R⁵ and R⁶ is independently selected from H, C₁-C₁₂ alkyl, C₅-C₁₀ aryl, and C₃-C₁₀ heterocyclic, wherein one or more carbons of said alkyl are optionally replaced by one or more diradicals selected from -O-, -S-, -S(O)-, -S(O)₂-, -NH-, -N(C₁-C₆) alkyl- and -C(O)- and said alkyl, aryl, and heterocyclic groups are optionally substituted by one or more substituents selected from the group T substituents;
R⁵ and R⁶ together with the nitrogen to which they are attached can form a 3- to 10-membered ring, in which one or more carbons are optionally replaced by one or more diradicals selected from -O-, -S-, -S(O)-, -S(O)₂-, -NH-, -N(C₁-C₆) alkyl- and -C(O)- and are optionally substituted by one or more substituents selected from the group T substituents;
R⁷ is selected from the group consisting of C₅-C₁₀ aryl, substituted C₅-C₁₀ aryl, 5- to 10- membered heteroaryl, substituted 5- to 10- membered heteroaryl and 3- to 10- membered heterocycloalkyl;
each of R⁸ and R⁹ is independently selected from the group consisting of C₁-C₁₂ alkenyl, C₁-C₁₂ alkynyl, C₅-C₁₀ aryl, C₃-C₈ cycloalkyl, 3- to 10- membered heterocycloalkyl and 5- to 10-membered heteroaryl, wherein said alkenyl, alkynyl, aryl, cycloalkyl, heterocycloalkyl and heteroaryl are optionally substituted by one or more substituents selected from the group T substituents;
R¹⁰ is -OCONR¹¹R¹²;
R¹¹ is H or C₁-C₆ alkyl;
R¹² is H, C₁-C₆ alkyl, -(CR¹³R¹⁴)ᵤ(C₃-C₁₀ cycloalkyl), -(CR¹³R¹⁴)ᵤ(C₅-C₁₀ aryl),-(CR¹³R¹⁴)ᵤ(3- to 10- membered heterocycloalkyl), (CR¹³R¹⁴)ᵤ(5- to 10-membered heteroaryl), wherein u is an integer from 0 to 10 and said aryl, cycloalkyl, heterocycloalkyl and heteroaryl in said R¹² group are optionally substituted by one or more substituents selected from the group T substituents;
R¹³ and R¹⁴ are independently H or C₁-C₆ alkyl;
each of R^{a} and R^{b} is independently selected from H, halo and C₁-C₆ alkyl;
R^{a} and R^{b} together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-,-N(C₁-C₆)alkyl- and -C(O)- and are optionally substituted by one or more substituents selected from the group T substituents;
(CR^{a}R^{b})ₙ is alkylene, wherein n is an integer ranging from 0 to 10, uninterrupted or interrupted by one or more diradicals selected from -O-, -S-, -S(O)-, -S(O)₂-, -NH-, -N(C₁-C₆)alkyl- and -C(O)- and optionally substituted by one or more substituents selected from the group T substituents; or a compound of formula I wherein C-3 is substituted by (=O) in place of the sugar group shown.

In preferred embodiments, R¹ is an alpha-branched C₃-C₈ alkyl, alkenyl, alkynyl, alkoxyalkyl or alkylthioalkyl group any of which may optionally be substituted by one or more hydroxyl groups; C₅-C₈ cycloalkylalkyl group wherein the alkyl group is an alpha-branched C₂-C₅ alkyl group; C₃-C₈ cycloalkyl or C₅-C₈ cycloalkenyl group, either of which may optionally be substituted by methyl or one or more hydroxyl or one or more C₁-C₄ alkyl groups or halo atoms; or a 3 to 6 membered oxygen or sulphur containing heterocyclic ring which may be saturated, or fully or partially unsaturated and which may optionally be substituted by one or more C₁-C₄ alkyl groups or halo atoms;
or R¹ is phenyl or C₆-C₁₂ aralkyl which is optionally substituted with at least one substituent selected from C₁-C₄ alkyl, C₁-C₄ alkoxy and C₁-C₄ alkylthio groups, halogen atoms, hydroxyl groups, trifluoromethyl, and cyano.

In further preferred embodiments, R¹ is Me, ethyl, n-butyl, MeS, EtS, cyclopropyl, cyclobutyl, cyclopentyl, or cyclohexyl.

In a further preferred embodiment, X¹ is -C(O)-.

In a further preferred embodiment, Ar is selected from phenyl, 2-methoxyphenyl, 4-methoxyphenyl, quinolin-4-yl, 7-methoxy-quinolin-4-yl, 4-phenyl-imidazol-1-yl, pyridin-4-yl, pyridin-3-yl, pyridin-2-yl, 4-pyridinyl-1H-imidazol-1-yl, imidazo(4,5-b)pyridin-3-yl, 2-phenyl-thiazol-5-yl, 2-pyridin-3-yl-thiazol-4-yl and benzimidazol-1-yl.

In a further preferred embodiment, B is selected from NH, NMe and CH₂, and R² is (CH₂)ₙAr. In a specific embodiment of this type, n is 3. Ar is preferably selected from quinolin-4-yl, 4-phenyl-1H-imidazol-1-yl, imidazo(4,5-b)pyridin-3-yl and 4-pyridinyl-1H-imidazol-1-yl.

In a further preferred embodiment, R¹ is ethyl, R³ is H, R¹⁰ is -OC(O)NH₂, and X¹ is-C(O)-. Among these compounds are especially preferred those wherein B is (CR^{aa}R^{bb})ₘ where m is 0 (i.e., B is a bond), and R² is (CH₂)₄-(5- to 10- membered heteroaryl). Further preferred within this latter group are those wherein heteroaryl in said R² group is 4-pyridinyl-1H-imidazol-1-yl.

In a further preferred embodiment, R¹ is ethyl, R³ is H, R¹⁰ is -OC(O)NH₂, X¹ is -C(O)-, and -B-R² is -NH(CH₂)₃-(5- to 10- membered heteroaryl). Preferred compounds within this group are those wherein heteroaryl in said R² is quinolin-4-yl.

In a further preferred embodiment, R² is H, B is (CR^{aa}R^{bb})ₘ where m is 0 (i.e., B is a bond), R¹ ethyl, R³ is H, X¹ is -C(O)-, and R¹⁰ is OC(O)NHR¹¹R¹². Preferred compounds within this group are those wherein R¹¹ is H and R¹² is -CH₂CH=CH-(quinolin-3-yl).

The invention also relates to a pharmaceutical composition for the treatment of a bacterial infection or a protozoa infection in a mammal, fish, or bird which comprises a therapeutically effective amount of a compound of formula I, or a pharmaceutically acceptable salt thereof, and a pharmaceutically acceptable carrier.

The invention also relates to a method of treating a bacterial infection or a protozoa infection in a mammal, fish, or bird which comprises administering to said mammal, fish or bird a therapeutically effective amount of a compound of formula I or a pharmaceutically acceptable salt thereof.

The term "treatment", as used herein, unless otherwise indicated, includes the treatment or prevention of a bacterial infection or protozoa infection as provided in the method of the present invention.

As used herein, unless otherwise indicated, the terms "bacterial infection(s)" and "protozoa infection(s)" include bacterial infections and protozoa infections that occur in mammals, fish and birds as well as disorders related to bacterial infections and protozoa infections that may be treated or prevented by administering antibiotics such as the compounds of the present invention. Such bacterial infections and protozoa infections, and disorders related to such infections, include the following: pneumonia, otitis media, sinusitus, bronchitis, tonsillitis, and mastoiditis related to infection by *Streptococcus pneumoniae, Haemophilus influenzae*, *Moraxella catarrhalis*, *Staphylococcus aureus,* or *Peptostreptococcus* spp.; pharynigitis, rheumatic fever, and glomerulonephritis related to infection by *Streptococcus pyogenes,* Groups C and G streptococci, *Clostridium diptheriae*, or *Actinobacillus haemolyticum;* respiratory tract infections related to infection by *Mycoplasma pneumoniae*, *Legionella pneumophila*, *Streptococcus pneumoniae*, *Haemophilus influenzae,* or *Chlamydia pneumoniae;* uncomplicated skin and soft tissue infections, abscesses and osteomyelitis, and puerperal fever related to infection by *Staphylococcus aureus,* coagulase-positive staphylococci (i.e., *S. epidermidis*, *S*. *hemolyticus*, etc.), *Streptococcus pyogenes*, *Streptococcus agalactiae,* Streptococcal groups C-F (minute-colony streptococci), viridans streptococci, *Corynebacterium minutissimum*, *Clostridium* spp., or *Bartonella henselae;* uncomplicated acute urinary tract infections related to infection by *Staphylococcus saprophyticus* or *Enterococcus* spp.; urethritis and cervicitis; and sexually transmitted diseases related to infection by *Chlamydia trachomatis*, *Haemophilus ducreyi*, *Treponema pallidum*, *Ureaplasma urealyticum,* or *Neiserria gonorrheae;* toxin diseases related to infection by S. *aureus* (food poisoning and Toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae*, *S*. *aureus, S*. *pneumoniae*, *S. pyogenes, H*. *influenzae*, or *Listeria* spp.; disseminated *Mycobacterium avium* complex (MAC) disease related to infection by *Mycobacterium avium,* or *Mycobacterium intracellulare;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis related to infection by *Helicobacter pylori* or *Chlamydia pneumoniae.* Bacterial infections and protozoa infections and disorders related to such infections that may be treated or prevented in animals include the following: bovine respiratory disease related to infection by *P. haem*., *P. multocida, Mycoplasma bovis,* or *Bordetella* spp.; cow enteric disease related to infection by *E*. *coli* or protozoa (i.e., coccidia, cryptosporidia, etc.); dairy cow mastitis related to infection by *Staph. aureus, Strep. uberis, Strep. agalactiae, Strep. dysgalactiae, Klebsiella* spp., *Corynebacterium,* or *Enterococcus* spp.; swine respiratory disease related to infection by *A. pleuro*., *P. multocida*, or *Mycoplasma* spp.; swine enteric disease related to infection by *E*. *coli*, *Lawsonia intracellularis*, *Salmonella,* or *Serpulina hyodyisinteriae;* cow footrot related to infection by *Fusobacterium* spp.; cow metritis related to infection by *E*. *coli*; cow hairy warts related to infection by *Fusobacterium necrophorum* or *Bacteroides nodosus;* cow pink-eye related to infection by *Moraxella bovis;* cow premature abortion related to infection by protozoa (i.e. neosporium); urinary tract infection in dogs and cats related to infection by *E. coli;* skin and soft tissue infections in dogs and cats related to infection by *Staph. epidermidis, Staph. intermedius, coagulase neg*. *Staph.* or *P. multocida;* and dental or mouth infections in dogs and cats related to infection by *Alcaligenes* spp., *Bacteroides* spp., *Clostridium* spp., *Enterobacter* spp., *Eubacterium, Peptostreptococcus*, *Porphyromonas,* or *Prevotella.* Other bacterial infections and protozoa infections and disorders related to such infections that may be treated or prevented in accord with the method of the present invention are referred to in J. P. Sanford et al., "The Sanford Guide To Antimicrobial Therapy," 26th Edition, (Antimicrobial Therapy, Inc., 1996).

The present invention also relates to a method of preparing the above compound of formula I, or a pharmaceutically acceptable salt thereof, which comprises treating a compound of the formula 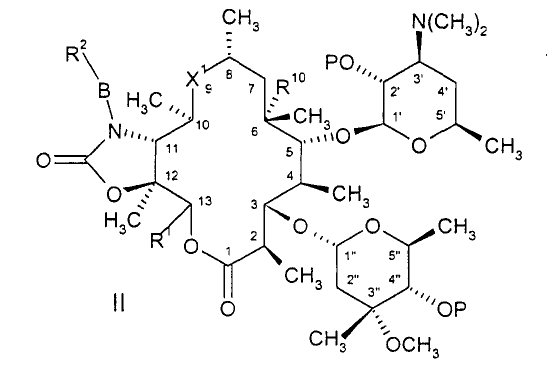 wherein X, R¹, and R² are as defined above, and where P is a hydroxy protecting group, to remove the hydroxy protecting group. Preferably, P is an acetyl group.

In a further aspect of the above process of preparing the compound of formula **I**, or a pharmaceutically acceptable salt thereof, the above compound of formula **II** is prepared by treating a compound of the formula 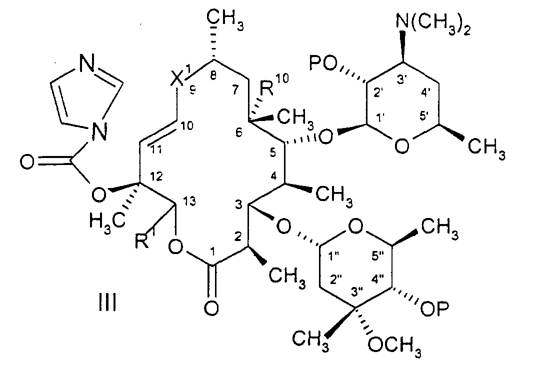 with R²-B-NH₂.

The term "Me", as used herein, unless otherwise indicated, refers to methyl.

The term "Et", as used herein, unless otherwise indicated, refers to ethyl.

The term "Pr", as used herein, unless otherwise indicated, refers to propyl.

The term "Ac", as used herein, unless otherwise indicated, refers to acetyl.

The term "hydroxy protecting group", as used herein, unless otherwise indicated, includes acetyl, benzyloxycarbonyl, and various hydroxy protecting groups familiar to those skilled in the art including the groups referred to in T. W. Greene, P. G. M. Wuts, "Protective Groups In Organic Synthesis," (J. Wiley & Sons, 1991).

The term "halo", as used herein, unless otherwise indicated, includes fluoro, chloro, bromo or iodo.

The term "alkyl", as used herein, unless otherwise indicated, includes saturated monovalent hydrocarbon radicals having straight, cyclic or branched moieties, or mixtures thereof. It is to be understood that where cyclic moieties are intended, at least three carbons in said alkyl must be present. Such cyclic moieties include cyclopropyl, cyclobutyl and cyclopentyl.

The term "alkenyl", as used herein, unless otherwise indicated, includes straight-chain or branched-chain mono- or poly-unsaturated aliphatic hydrocarbon radicals containing at least one carbon-carbon double bond. Examples of alkenyl radicals include, but are not limited to, ethenyl, E- and Z-propenyl, isopropenyl, E- and Z-butenyl, E- and Z-isobutenyl, E- and Z-pentenyl, E- and Z-hexenyl, E,E-, E,Z-, Z,E- and Z,Z-hexadienyl and the like.

The term "alkynyl", as used herein, unless otherwise indicated, includes straight-chain or branched-chain mono- or poly-unsaturated aliphatic hydrocarbon radicals containing at least one carbon-carbon triple bond. Examples of alkynyl radicals include, but are not limited to, ethynyl, propynyl, isopropynyl, butynyl, isobutynyl, pentynyl, hexynyl and the like.

The term "alkoxy", as used herein, unless otherwise indicated, includes -O-alkyl groups wherein alkyl is as defined above.

The term "aryl", as used herein, unless otherwise indicated, includes an organic radical derived from an aromatic hydrocarbon by the removal of one hydrogen. Examples of aryl radicals include, but are not limited to, phenyl, naphthyl, indenyl, indanyl, azulenyl, fluorenyl, anthracenyl and the like.

The term "3- to 10-membered heterocyclic", as used herein, unless otherwise indicated, includes aromatic and non-aromatic heterocyclic groups containing one or more heteroatoms, each selected from O, S and N, wherein each heterocyclic group has from 3 to 10 atoms in its ring system. Non-aromatic heterocyclic groups include groups having only 3 atoms in their ring system, but aromatic heterocyclic groups must have at least 5 atoms in their ring system. The heterocyclic groups include benzo-fused ring systems and ring systems substituted with one or more oxygen or nitrogen atoms. The heterocyclic groups also include partially unsaturated or fully saturated 3- to 10-membered ring systems, e.g., single rings of 3 to 8 atoms in size and bi- or tricyclic ring systems, including aromatic 6-membered aryl or heteroaryl rings fused to a non-aromatic ring. An example of a 4-membered heterocyclic group is azetidinyl (derived from azetidine). An example of a 5-membered heterocyclic group is thiazolyl, and an example of a 10-membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached).

The term "protecting group" refers to a suitable chemical group that may be attached to a functional group and removed at a later stage to reveal the intact functional group. Examples of suitable protecting groups for various functional groups are described in T.W. Greene and P.G.M Wuts, Protective Groups in Organic Synthesis, 2d Ed., John Wiley and Sons (1991); L. Fieser and M. Fieser, Fieser and Fieser's Reagents for Organic Synthesis, John Wiley and Sons (1994); and L. Paquette, ed. Encyclopedia of Reagents for Organic Synthesis, John Wiley and Sons (1995).

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds of are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include an amino moiety may form pharmaceutically acceptable salts with various amino acids, in addition to the acids mentioned above.

Those compounds of the present invention that are acidic in nature are capable of forming base salts with various pharmacologically acceptable cations. Examples of such salts include the alkali metal or alkaline earth metal salts and, particularly, the calcium, magnesium, sodium and potassium salts of the compounds of the present invention.

Certain compounds of the present invention may have asymmetric centers and therefore exist in different enantiomeric and diastereomic forms. This invention relates to the use of all optical isomers and stereoisomers of the compounds of the present invention, and mixtures thereof, and to all pharmaceutical compositions and methods of treatment that may employ or contain them.

The present invention includes the compounds of the present invention, and the pharmaceutically acceptable salts thereof, wherein one or more hydrogen, carbon or other atoms are replaced by isotopes thereof. Such compounds may be useful as research and diagnostic tools in metabolism pharmacokinetic studies and in binding assays.

As used herein, the compounds of this invention, including the compounds of formula I, are defined to include pharmaceutically acceptable derivatives or prodrugs thereof. A "pharmaceutically acceptable derivative or prodrug" means any pharmaceutically acceptable salt, ester, salt of an ester or other derivative of a compound of this invention that, upon administration to a recipient, is capable of providing (directly or indirectly) a compound of this invention or a metabolite or residue thereof. Particularly favored derivatives and prodrugs are those that increase the bioavailability of the compounds of this invention when such compounds are administered to a patient (e.g., by allowing an orally administered compound to be more readily absorbed into the blood), enhance delivery of the parent compound to a given biological compartment, increase solubility to allow administration by injection, alter metabolism or alter rate of excretion.

Compounds of the invention may exist in tautomeric form. All tautomers of the compounds of formula I are included in the invention.

Compounds of formula I can be converted into prodrugs through, for example, free amino, amido, hydroxy or carboxylic groups. Examples of such prodrugs include compounds wherein an amino acid residue, or a polypeptide chain of two or more (e.g., two, three or four) amino acid residues is covalently joined through an amide or ester bond to a free amino, hydroxy or carboxylic acid group of a compound of formula I. The amino acid residues include but are not limited to the 20 naturally occurring amino acids commonly designated by three letter symbols and also include 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone.

Additional types of prodrugs are also encompassed. For instance, free carboxyl groups can be derivatized as amides or alkyl esters. The amide and ester moieties may incorporate groups including but not limited to ether, amine and carboxylic acid functionalities. Free hydroxy groups may be derivatized using groups including but not limited to hemisuccinates, phosphate esters, dimethylaminoacetates and phosphoryloxymethyloxycarbonyls, as outlined in D. Fleisher et al., Advanced Drug Delivery Reviews, vol. 19, p. 115 (1996). Carbamate prodrugs of hydroxy and amino groups are also included, as are carbonate prodrugs and sulfate esters of hydroxy groups. Derivatization of hydroxy groups as (acyloxy)methyl and (acyloxy)ethyl ethers wherein the acyl group may be an alkyl ester, optionally substituted with groups including but not limited to ether, amine and carboxylic acid functionalities, or where the acyl group is an amino acid ester as described above, are also encompassed. Prodrugs of this type are described in R.P. Robinson et al., J. Medicinal Chemistry, vol. 39, p. 10 (1996).

The compounds of this invention also include pharmaceutically acceptable salts of the compounds of formula I. The term "pharmaceutically acceptable salt(s)", as used herein, unless otherwise indicated, includes salts of acidic or basic groups that may be present in the compounds of the present invention.

### Detailed Description of the Invention

All patents, publications, and patent applications referred to herein are hereby incorporated by reference in their entireties.

The compounds of the present invention may be prepared according to Scheme 1. In the Scheme, unless otherwise indicated, all of the substituents are as defined above.

The starting materials used in the present invention may require proper functional group protection before various modifications can take place, and deprotection after desired modifications are complete. Hydroxyl groups are generally protected as acetates or Cbz carbonates. The relative reactivity of various hydroxyl groups in the macrolide molecules of the general type claimed in this invention has been well established. Such differences in reactivity permit selective modification of different parts of the compounds of this invention.

The synthetic process for preparation of compounds of formulae **5a-5c** of the invention employs a 6-carbamoyl erythromycin A derivative **1**. Compounds of formula **1** and their syntheses are disclosed in US patent 4,826,820. The resulting compound is treated with DBU (1,8-diazabicyclo[5.4.0]undec-7-ene) and CDI (carbonyl diimidazole) to generate the 12-imidazolecarbonyloxy derivative **3.** Treatment of this material with ammonia, hydrazine, or hydroxylamine generates compound **4a, 4b,** or **4c,** respectively. Removal of the protecting groups provides compounds **5a, 5b,** or **5c.**

In particular, the compound of formula **1** is treated with **2** equivalents of acetic anhydride in the presence of a catalytic amount of 4-N,N,-dimethylaminopyridine in an inert solvent such as CH₂Cl₂ at 0° to 50°C, preferably room temperature for 2 to 24 hours, preferably 12 hours (Scheme 1, Step 1). The diacetyl derivative of formula **2** is then treated with 1-2 eq of DBU and 1 eq of CDI in an inert solvent such as CH₂Cl₂ at room temperature for 4 to 24 hours, preferably 12 hours (Scheme 1, Step 2) to obtain the 12-imidazolecarbonyloxy derivative of formula **3.** The compound of formula **3** is the treated with 1-3 eq of ammonia, hydrazine or hydroxylamine in acetonitrile at 0° to 100°C, preferably 80° for 2-24 hours, preferably 12 hours (Scheme 1, Step 3) to obtain the compound of formula **4.** The acetyl groups are removed from the compound of formula **4** by warming in methanol at room temperature to 80°C, preferably 50°C for 2-24 hours, preferably 12 hours (Scheme 1, Step 4) to obtain the compound of formula **5.**

Compounds of formula **1** containing an oxime at the 9 position can be obtained by converting a corresponding oxime lacking a carbamoyl at carbon 6, e.g., such as is described in US patent 4,990,602, to the corresponding cabamoyl **6** using methods known in the art, and to those described in US patent 4,826,820.

The compound of formula **1,** can also be obtained by converting a 6-carbamoyl-11,12-diol of formula **7** (R²=OCONH₂) to the cyclic carbonate of formula **1** by treatment with ethyleneglycol carbonate and K₂CO₃. 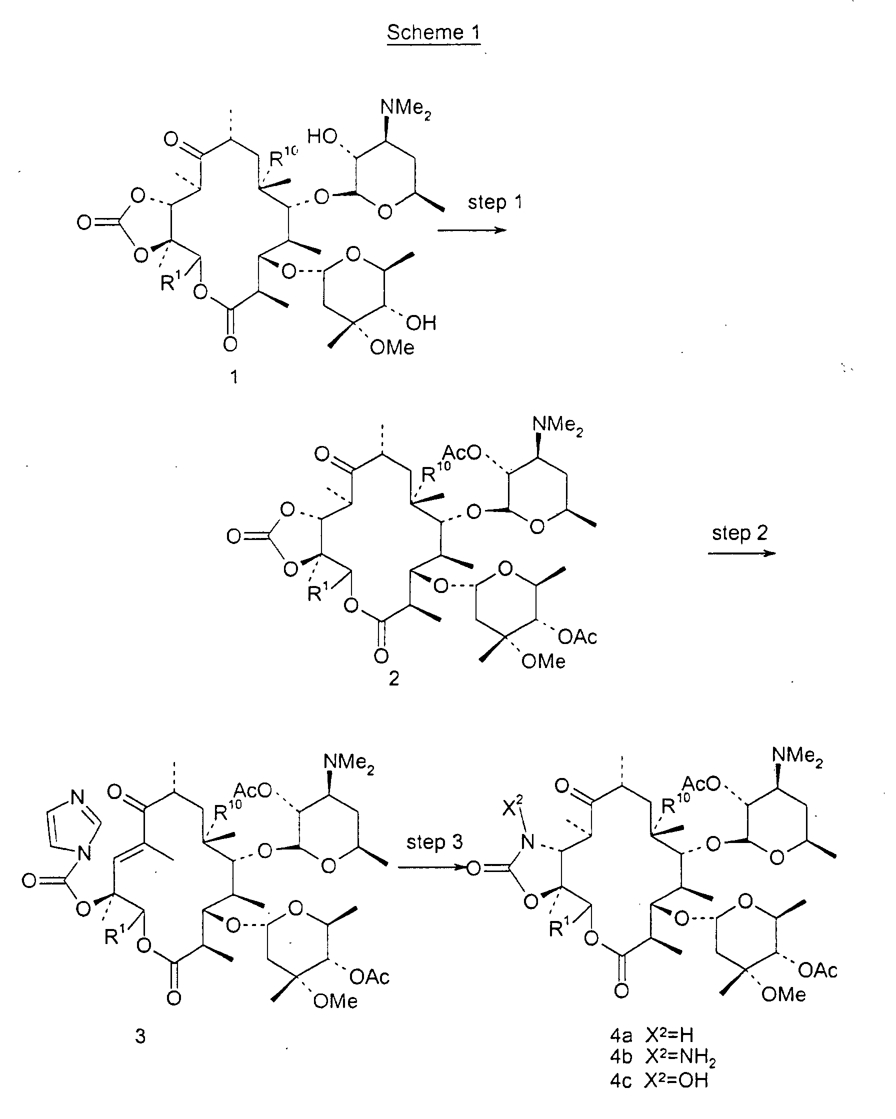 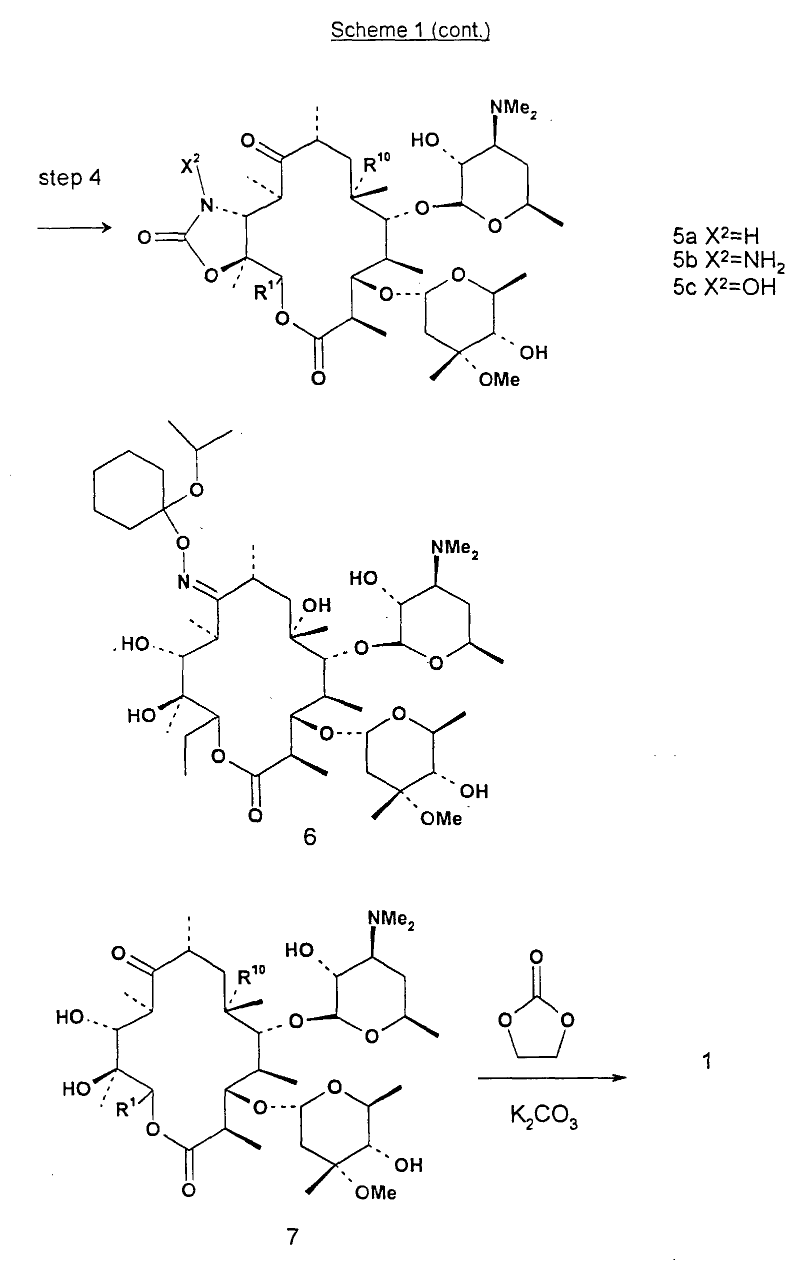 In Scheme 2, compound **2** from Scheme 1 is treated with anhydrous HCI at 0°C to room temperature, preferably room temperature for 1 to 24 hours., preferably 12 hours, to prepare compound **3.** The resulting compound is then treated with DMSO and EDCI (1-(3-dimethylaminopropyl)-3-ethylcarbodiimide hydrochloride) at 0°C to room temperature, preferably room temperature, for 1 to 24 hours, preferably 12 hours, to generate compound **8.** Other oxidizing agents such as DMSO-oxalyl chloride or Dess-Martin periodinane reagent can also be employed. Compound **8** is treated with 1-2 equivalent of DBU in an inert solvent at 0 to 40°C, preferably 40°C, for 1 to 24 hours, preferably 12 hours. The resulting alcohol is then treated with NaH and carbonyl diimidazole (CDI) in THF at 0°C to room temperature, to generate compound **10.** Compound **10** is treated with R²-B-NH₂ in acetonitrile at room temperature to 80°C, preferably 80°C, for 1 to 24 hours, preferably 12 hours. The resulting compound is then treated with MeOH at room temperature to 64°C, preferably 50°C, for 1 to 24 hours, preferably 12 hours, to remove the 2' acetyl group to give compound **11**. 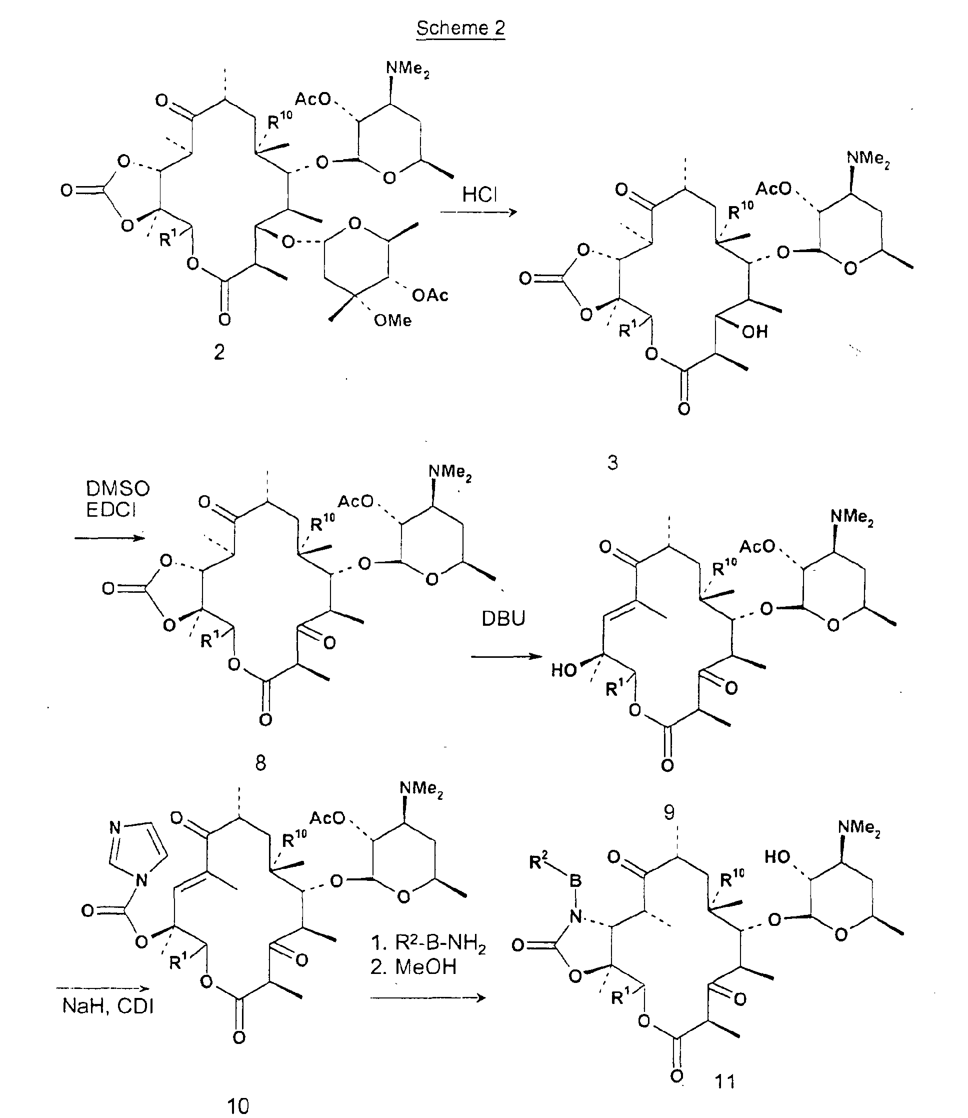 In Scheme 3, compound **4b** from Scheme 1 is treated under reductive amination conditions, that is, with an appropriate aldehyde with sodium cyanoborohydride, to give compound **12.** Compound **12** is first treated with anhydrous HCI to remove the cladinose sugar, and then with an oxidizing agent, as described above, to prepare compound **13.**

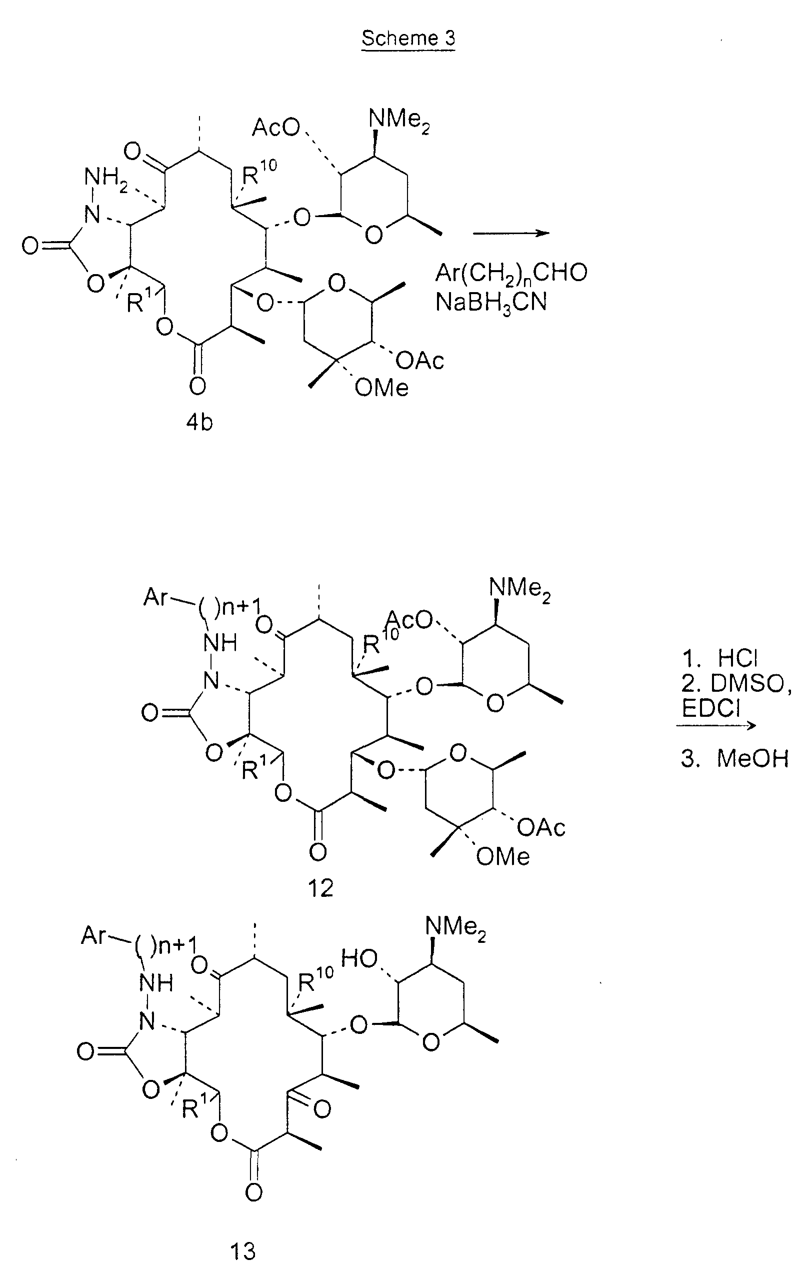

In Scheme 4 compound **7**, e.g., erythromycin A, is treated with ethylene carbonate and K₂CO₃ in ethyleneglycol dimethyl ether at 50 to 85 °C, preferably 85°C, for 6 to 24 hours, preferably 24 hours, to give compound **14**. This is treated with acetic anhydride in THF at 0°C to room temperature, preferably 0°C, for 1 hr to 12 hours, preferably 12 hours to give compound **15.** Compound **15** is then treated with trichloroacetyl isocyanate in dichloromethane at -10°C to room temperature, preferably 0°C, for 5 min to 5 hours, preferably 5 min. The resulting intermediates are treated with excess methanol to give compounds **16** and **17.**

Tricarbamate **17** is heated in aqueous acetonitrile at 50 to 80°C, preferably 80°C, for 3 to 24 hours, preferably 12 hours, to give compound **18.** The cladinose sugar is cleaved as described above and the resulting alcohol is oxidized to ketone **19.** This is then treated with excess base such as NaH and R-X³ (X³ is halogen, mesylate, tosylate, or other leaving group). The resulting intermediates are treated with MeOH to remove the C2' acetyl group to give compound **20** or **21.** Dicarbamate **16** is treated with anhydrous HCI as described above to cleave the cladinose sugar and oxidized to give compound **22.** This is further treated with NaH and CDI to give compound **10,** which can be utilized as shown in Scheme 2. 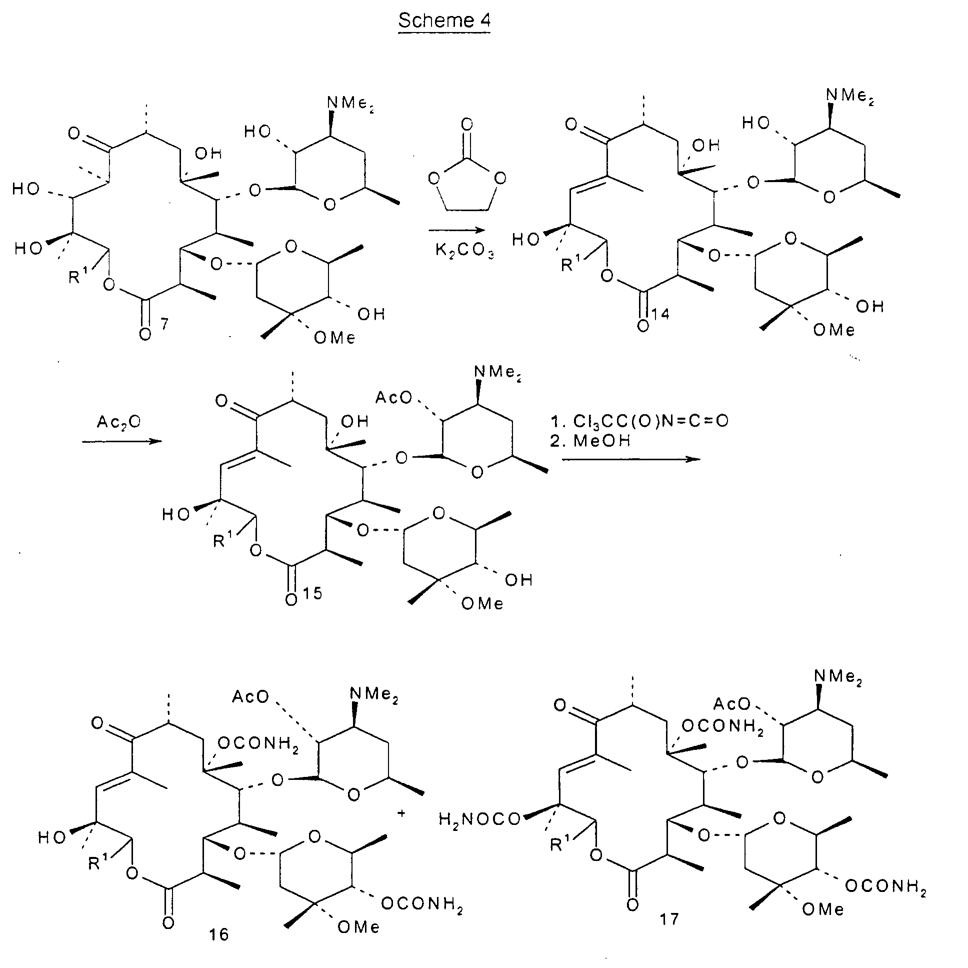 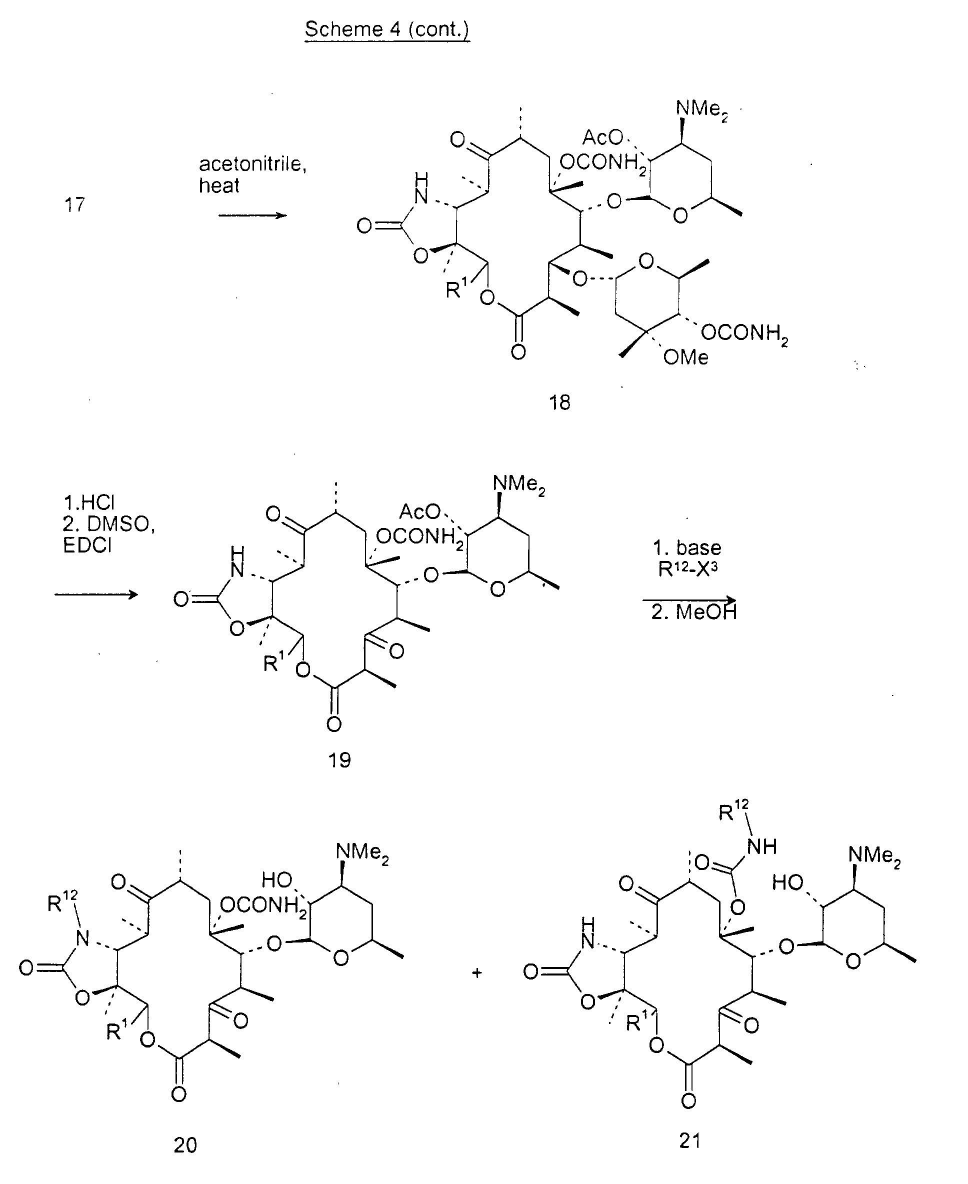 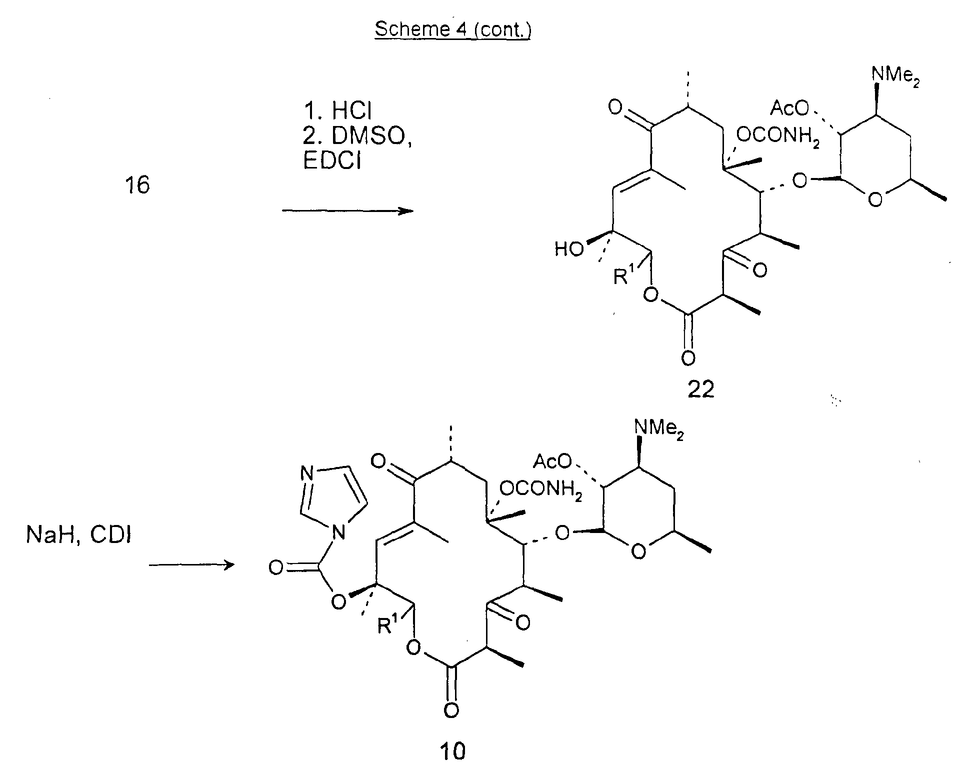 Compounds of the present invention wherein R¹ is other than ethyl may be prepared using starting materials obtained as described, e.g., in WO 98/01546, published January 15, 1998; WO 98/01571, published January 15, 1998; WO 00/0500, published January 6, 2000; WO 98/54308, published December 3, 1998; and WO 00/00618, published January 6, 2000.

The compounds of the present invention may have asymmetric carbon atoms and therefore exist in different enantiomeric and diastereomeric forms. Diastereomeric mixtures can be separated into their individual diastereomers on the basis of their physical chemical differences by methods known to those skilled in the art, for example, by chromatography or fractional crystallization. The use of all such isomers, including diastereomer mixtures and pure enantiomers, are considered to be part of the present invention.

The compounds of the present invention that are basic in nature are capable of forming a wide variety of different salts with various inorganic and organic acids. Although such salts must be pharmaceutically acceptable for administration to mammals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired salt can also be precipitated from a solution of the free base in an organic solvent by adding to the solution an appropriate mineral or organic acid.

Those compounds of the present invention that are acidic in nature are capable of forming base salts with various cations. For compounds that are to be administered to mammals, fish or birds such salts must be pharmaceutically acceptable. Where a pharmaceutically acceptable salt is required, it may be desirable to initially isolate the compound of the present invention from the reaction mixture as a pharmaceutically unacceptable salt and then simply convert the latter to a pharmaceutically acceptable salt in a process analogous to that described above relating to the conversion of pharmaceutically unacceptable acid addition salts to pharmaceutically acceptable salts. Examples of base salts include the alkali metal or alkaline-earth metal salts and particularly the sodium, amine and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium, calcium, magnesium, various amine cations, etc. These salts can easily be prepared by treating the corresponding acidic compounds with an aqueous solution containing the desired pharmacologically acceptable bases with cations such as sodium, potassium, calcium, magnesium, various amine cations, etc., and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The present invention includes all isotopically labelled forms of the compounds of formula I, and pharmaceutically acceptable salts and prodrugs thereof. Such isotopically labelled compounds are useful as research or diagnostic tools. The isotopically-labelled compounds and pharmaceutically acceptable salts thereof are identical to those of formula I but for the fact that one or more atoms are replaced by an atom having an atomic mass or mass number different from the atomic mass or mass number usually found in nature. Examples of isotopes that can be incorporated into compounds of this invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³⁵S, ¹⁸F and ³⁶Cl, respectively. Certain isotopically labelled compounds of the present invention, such as those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Substitution with heavier isotopes such as deuterium, i.e., ²H, can afford certain therapeutic advantages resulting from greater metabolic stability, for example, increased *in vivo* half-life or reduced dosage requirements, and hence may be preferred in some circumstances. Isotopically labelled compounds of formula I of this invention and prodrugs thereof can generally be prepared by carrying out the procedures disclosed in the Scheme(s) and/or in the Example(s) below and substituting a readily available isotopically labelled reagent for a non-isotopically labelled reagent.

The antibacterial and antiprotozoa activity of the compounds of the present invention against bacterial and protozoa pathogens is demonstrated by the compound's ability to inhibit growth of defined strains of human (Assay I) or animal (Assays II and III) pathogens.

### Assay I

Assay I, described below, employs conventional methodology and interpretation criteria and is designed to provide direction for chemical modifications that may lead to compounds that circumvent defined mechanisms of macrolide resistance. In Assay I, a panel of bacterial strains is assembled to include a variety of target pathogenic species, including representatives of macrolide resistance mechanisms that have been characterized. Use of this panel enables the chemical structure/activity relationship to be determined with respect to potency, spectrum of activity, and structural elements or modifications that may be necessary to obviate resistance mechanisms. Bacterial pathogens that comprise the screening panel are shown in the table below. In many cases, both the macrolide-susceptible parent strain and the macrolide-resistant strain derived from it are available to provide a more accurate assessment of the compound's ability to circumvent the resistance mechanism. Strains that contain the gene with the designation of *ermA/ermB/ermC* are resistant to macrolides, lincosamides, and streptogramin B antibiotics due to modifications (methylation) of 23S rRNA molecules by an Erm methylase, thereby generally prevent the binding of all three structural classes. Two types of macrolide efflux have been described; *msrA* encodes a component of an efflux system in staphylococci that prevents the entry of macrolides and streptogramins while *mefA/E* encodes a transmembrane protein that appears to efflux only macrolides. Inactivation of macrolide antibiotics can occur and can be mediated by either a phosphorylation of the 2'-hydroxyl (*mph*) or by cleavage of the macrocyclic lactone (esterase). The strains may be characterized using conventional polymerase chain reaction (PCR) technology and/or by sequencing the resistance determinant. The use of PCR technology in this application is described in J. Sutcliffe et al., "Detection Of Erythromycin-Resistant Determinants By PCR", Antimicrobial Agents and Chemotherapy, 40(11), 2562-2566 (1996). The assay is performed in microtiter trays and interpreted according to Performance Standards for Antimicrobial Disk Susceptibility Tests - Sixth Edition; Approved Standard, published by The National Committee for Clinical Laboratory Standards (NCCLS) guidelines; the minimum inhibitory concentration (MIC) is used to compare strains. Compounds are initially dissolved in dimethylsulfoxide (DMSO) as 40 mg/ml stock solutions.

| Strain Designation | Macrolide Resistance Mechanism(s) |
|---|---|
| *Staphylococcus aureus* 1116 | susceptible parent |
| *Staphylococcus aureus* 1117 | *ermB* |
| *Staphylococcus aureus* 0052 | susceptible parent |
| *Staphylococcus aureus* 1120 | *ermC* |
| *Staphylococcus aureus* 1032 | *msrA, mph,* esterase |
| *Staphylococcus hemolyticus* 1006 | *msrA, mph* |
| *Streptococcus pyogenes* 0203 | susceptible parent |
| *Streptococcus pyogenes* 1079 | *ermB* |
| *Streptococcus pyogenes* 1062 | susceptible parent |
| *Streptococcus pyogenes* 1061 | *ermB* |
| *Streptococcus pyogenes* 1064 | *ermB* |
| *Streptococcus agalactiae* 1024 | susceptible parent |
| *Streptococcus agalactiae* 1023 | *ermB* |
| *Streptococcus pneumoniae* 1016 | susceptible |
| *Streptococcus pneumoniae* 1046 | *ermB* |
| *Streptococcus pneumoniae* 1095 | *ermB* |
| *Streptococcus pneumoniae* 1175 | *mefE* |
| *Streptococcus pneumoniae* 0085 | susceptible |
| *Haemophilus influenzae* 0131 | susceptible |
| *Moraxella catarrhalis* 0040 | susceptible |
| *Moraxella catarrhalis* 1055 | erythromycin intermediate resistance |
| *Escherichia coli* 0266 | susceptible |

Assay II is utilized to test for activity against *Pasteurella multocida* and Assay III is utilized to test for activity against *Pasteurella haemolytica.*

### Assay II

This assay is based on the liquid dilution method in microliter format. A single colony of *P. multocida* (strain 59A067) is inoculated into 5 ml of brain heart infusion (BHI) broth. The test compounds are prepared by solubilizing 1 mg of the compound in 125 µl of dimethylsulfoxide (DMSO). Dilutions of the test compound are prepared using uninoculated BHI broth. The concentrations of the test compound used range from 200 µg/ml to 0.098 µg/ml by two-fold serial dilutions. The *P. multocida* inoculated BHI is diluted with uninoculated BHI broth to make a 10⁴ cell suspension per 200 µl. The BHI cell suspensions are mixed with respective serial dilutions of the test compound, and incubated at 37°C for 18 hours. The minimum inhibitory concentration (MIC) is equal to the concentration of the compound exhibiting 100% inhibition of growth of P. multocida as determined by comparison with an uninoculated control.

### Assay III

This assay is based on the agar dilution method using a Steers Replicator. Two to five colonies isolated from an agar plate are inoculated into BHI broth and incubated overnight at 37°C with shaking (200 rpm). The next morning, 300 µl of the fully grown *P. haemolytica* preculture is inoculated into 3 ml of fresh BHI broth and is incubated at 37°C with shaking (200 rpm). The appropriate amounts of the test compounds are dissolved in ethanol and a series of two-fold serial dilutions are prepared. Two ml of the respective serial dilution is mixed with 18 ml of molten BHI agar and solidified. When the inoculated *P*. *haemolytica* culture reaches 0.5 McFarland standard density, about 5 µl of the *P. haemolytica* culture is inoculated onto BHI agar plates containing the various concentrations of the test compound using a Steers Replicator and incubated for 18 hours at 37°C. Initial concentrations of the test compound range from 100-200 µg/ml. The MIC is equal to the concentration of the test compound exhibiting 100% inhibition of growth of *P. haemolytica* as determined by comparison with an uninoculated control.

The in vivo activity of the compounds of formula (I) can be determined by conventional animal protection studies well known to those skilled in the art, usually carried out in mice.

Mice are allotted to cages (10 per cage) upon their arrival, and allowed to acclimate for a minimum of 48 hours before being used. Animals are inoculated with 0.5 ml of a 3 x 10³ CFU/ml bacterial suspension (*P*. *multocida* strain 59A006) intraperitoneally. Each experiment has at least 3 non-medicated control groups including one infected with 0.1X challenge dose and two infected with 1X challenge dose; a 10X challenge data group may also be used. Generally, all mice in a given study can be challenged within 30-90 minutes, especially if a repeating syringe (such as a Cornwall® syringe) is used to administer the challenge. Thirty minutes after challenging has begun, the first compound treatment is given. It may be necessary for a second person to begin compound dosing if all of the animals have not been challenged at the end of 30 minutes. The routes of administration are subcutaneous or oral doses. Subcutaneous doses are administered into the loose skin in the back of the neck whereas oral doses are given by means of a feeding needle. In both cases, a volume of 0.2 ml is used per mouse. Compounds are administered 30 minutes, 4 hours, and 24 hours after challenge. A control compound of known efficacy administered by the same route is included in each test. Animals are observed daily, and the number of survivors in each group is recorded. The *P. multocida* model monitoring continues for 96 hours (four days) post challenge.

The PD₅₀ is a calculated dose at which the compound tested protects 50% of a group of mice from mortality due to the bacterial infection which would be lethal in the absence of drug treatment.

The compounds of formula 1, and the pharmaceutically acceptable salts, prodrugs, tautomers, and solvates thereof (hereinafter "the active compounds"), may be administered through oral, parenteral, topical, or rectal routes in the treatment of bacterial and protozoa infections. In general, these compounds are most desirably administered in dosages ranging from about 0.2 mg per kg body weight per day (mg/kg/day) to about 200 mg/kg/day in single or divided doses (i.e., from 1 to 4 doses per day), although variations will necessarily occur depending upon the species, weight and condition of the subject being treated and the particular route of administration chosen. However, a dosage level that is in the range of about 4 mg/kg/day to about 50 mg/kg/day is most desirably employed. Variations may nevertheless occur depending upon the species of mammal, fish or bird being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds may be administered alone or in combination with pharmaceutically acceptable carriers or diluents by the routes previously indicated, and such administration may be carried out in single or multiple doses. More particularly, the active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active compound may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques will known to those skilled in the art.

Additionally, it is also possible to administer the active compounds of the present invention topically and this may be done by way of creams, jellies, gels, pastes, patches, ointments and the like, in accordance with standard pharmaceutical practice.

For administration to animals other than humans, such as cattle or domestic animals, the active compounds may be administered in the feed of the animals or orally as a drench composition.

The active compounds may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoylresidues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The following Examples further illustrate the method of the present invention. It is to be understood that the present invention is not limited to the specific details of the Examples provided below.

### Example 1

### 6, 4"-Biscarbamoyl-10,11-dehydro-erythromycin A (Scheme 4, Compound 16 wherein R¹ is ethyl)

To a solution of compound **15** wherein R¹ is ethyl (54 mg, 0.072 mmol) in 1 mL of methylene chloride, was added at 0 °C trichloroacetyl isocyanate (17.2 µL, 0.144 mmol). The mixture was stirred at this temperature for 30 min. and the solvent evaporated. The residue was dissolved in 5 mL of methanol and 100 µL of water and the solution stirred at room temperature overnight. The residue obtained after evaporation of methanol and water was chromatographed on silica gel TLC (4.5 % MeOH-0.5% NH₄OH-CH₂Cl₂) to give 39 mg (69%) of the title compound; MS 802 (M+1).

### Example 2

### 6, 12, 4"-Triscarbamoyl-10,11-dehydro-erythromycin A (Scheme 4, compound 17 wherein R¹ is ethyl)

To a solution of compound **15** (47 mg, 0.062 mmol) in **1** mL of methylene chloride was added at 0 °C trichloroacetyl isocyanate (22.2 µL, 0.188 mmol). The mixture was stirred at this temperature for 30 min. and the solvent evaporated. The residue was dissolved in 5 mL of methanol and 100 µL of water, and the solution stirred at room temperature overnight. The residue obtained after evaporation of methanol and water was chromatographed on silica gel TLC (4.5 % MeOH-0.5% NH₄OH-CH₂Cl₂) to give 29 mg (55%) of the title compound; MS 845 (M+1).

### Example 3

### 11-Deoxy-11-(carboxyamino)-6, 4"-biscarbamoyl-erythromycin A (Scheme 4, compound 18 wherein R¹ is ethyl)

The triscarbamoyl derivative (29 mg) obtained in Example 2 was dissolved in 2 mL of acetonitrile and 0.3 mL of water and heated at 80 °C overnight. The residue obtained after evaporation of the solvent was purified by silica gel TLC (10% MeOH-1% NH₄OH-CH₂Cl₂) to give 9 mg of the title compound; MS 845 (M+1).

## Claims

1. A compound of the formula 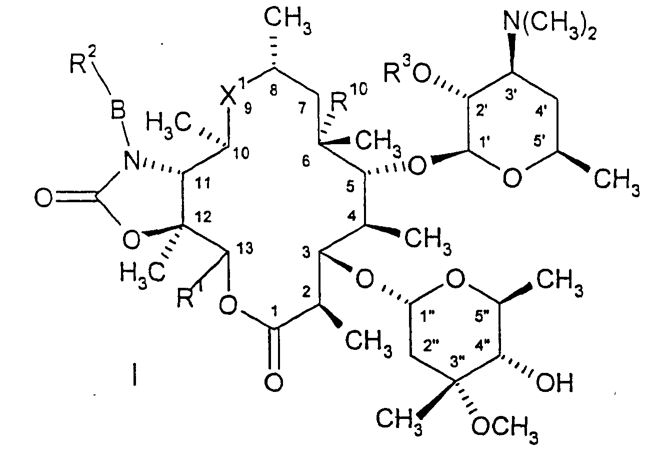 or a pharmaceutically acceptable salt, prodrug, tautomer, or solvate, thereof, wherein:
X¹ is selected from -C(O)-, -CH(NR⁵R⁶)-, -CHR⁵NR⁶-, -NR⁶CHR⁵-, -C(=NR⁵)- and-C(=N-OR⁵)-, wherein the first dash of each of the foregoing X¹ groups is attached to the C-10 carbon of the compound of formula I and the last dash of each group is attached to the C-8 carbon of the compound of formula I;
B is selected from O, (CR^{aa}R^{bb})ₘ, SO₂, and NR⁴, wherein m is 0 or 1;
R^{aa} and R^{bb} are independently selected from H and C₁-C₆ alkyl;
R^{aa} and R^{bb} together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
when B is NR⁴, B and R² together with the nitrogen to which they are attached can form a 3- to 10-membered ring wherein one or more carbons of said ring are optionally replaced by one or more diradicals selected from -O-, -S-, -S(O)-, -S(O)₂-, -NH-, -N(C₁-C₆) alkyl- and -C(O)- and are optionally substituted by one or more substituents selected from the group T substituents;
when B is NR⁴, B and R² together with the nitrogen to which they are attached can form -N=C(R⁴)(CR^{a}R^{b})ₙAr, wherein n is an integer ranging from 0 to 10;
B, R² and X¹ can be taken together;
when taken together, B, R² and X¹ taken with their intervening atoms form an additional two rings having one of the following structures: 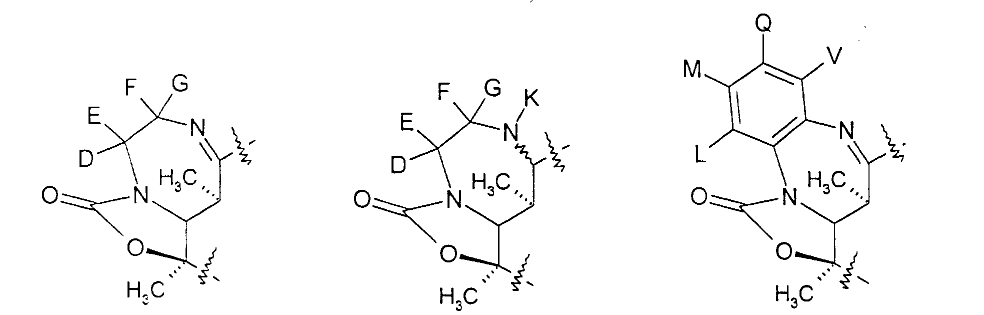 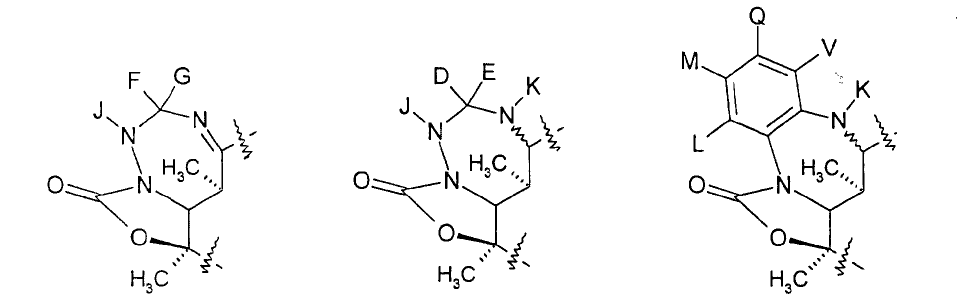 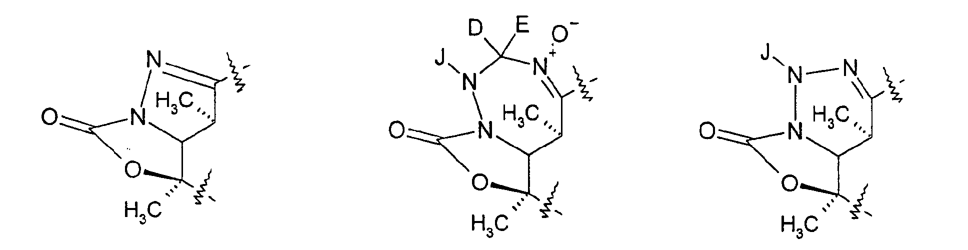 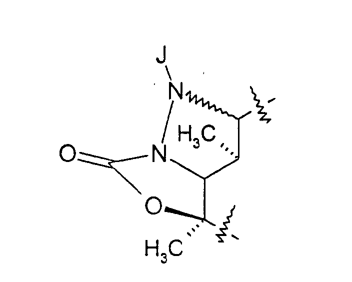 wherein:
each of D, E, F and G is independently selected from H, halo, C₁-C₁₂ alkyl, C₃-C₁₀ alkenyl, C₃-C₁₀ alkynyl and (CR^{a}R^{b})ₙAr, wherein n is an integer ranging from 0 to 10, wherein one or more carbons of said alkyl are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
D and E or F and G together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
each of J, J¹ and K is independently selected from C(O)R⁵, C(O)NR⁵R⁶, C(O)OR⁵, (CR^{a}R^{b})ₙAr, O(CR^{a}R^{b})ₙAr and N(CR^{a}R^{b})ₙAr; wherein n is an integer ranging from 0 to 10;
each of L, M, Q and V is independently selected from H and the group T substituents;
one or two carbons of the phenyl ring in which L, M, Q and V are attached can be replaced with nitrogen and L, M, Q, and V do not indicate any substituent where attached to nitrogen;
R¹ is C₁-C₂₀ alkyl, C₁-C₂₀ alkyl, C₆-C₁₀ aryl, or C₇-C₁₄ aralkyl, wherein one or more carbons of said alkyl are optionally replaced by one or more diradicals selected from -O-, -S-, -S(O)-, -S(O)₂-, -NH-, -N(C₁₋C₆)alkyl- and -C(O)- and are optionally substituted by one or more substituents selected from the group T substituents;
Ar is independently a 3- to 10-membered heterocyclic or C₅-C₁₀ aryl, wherein said heterocyclic and aryl groups are optionally substituted by one or more substituents independently selected from the group T substituents;
T substituents are selected from the group consisting of:
(a) nitro;
(b) halo;
(c) hydroxy;
(d) N₃;
(e) CN;
(f) CHO;
(g) C₁-C₁₀ alkoxy;
(h) C₁-C₃ alkoxy-C₁-C₃ alkoxy;
(i) oxo;
(j) C₁-C₁₀ alkanoyl;
(k) C₁-C₁₀ alkyl;
(l) C₁-C₁₂ alkyl substituted with an aromatic 5- to 10-membered heterocyclic;
(m) C₁-C₆ alkyl substituted with O-SO₂;
(n) C₂-C₁₀ alkenyl;
(o) C₂-C₁₀ alkynyl;
(p) C₃-C₁₀ cycloalkyl;
(q) substituted* C₃-C₁₀ cycloalkyl;
(r) 3- to 10-membered heterocyclic;
(s) substituted* 3- to 10-membered heterocyclic;
(t) C₅-C₁₀aryl;
(u) substituted* C₅-C₁₀ aryl;
(v) tri C₁-C₆ alkylsilyl;
(w) -C(O)R⁵;
(x) -C(O)₂R⁷;
(y) -C(O)OR⁵;
(z) -OC(O)R⁵;
(aa) -C(O)NR⁵R⁶;
(bb) -OC(O)NR⁵R⁶;
(cc) -O(C)OR⁵;
(dd) -NR⁵R⁶;
(ee) -NR⁸R⁹;
(ff) -NHC(O)R⁵;
(gg) -NHC(O)NR⁵R⁶;
(hh) =N-O-R⁵;
(ii) =N-NR⁵R⁶;
(jj) =N-NR⁸R⁹;
(kk) =N-R⁵;
(II) =N-R⁷;
(mm) =N-NHC(O)R⁵;
(nn) =N-NHC(O)NR⁵R⁶;
(oo) -C=N;
(pp) -S(O)ₙ, wherein n is 0, 1 or 2;
(qq) -S(O)ₙR⁵, wherein n is 0, 1 or 2;
(rr) -O-S(O)ₙR⁵, wherein n is 0, 1 or 2; and
(ss) -SO₂NR⁵R⁶;
wherein substituted* groups in this list can be substituted with T substituents;
R² is selected from H, C₁-C₁₂ alkyl, C₃-C₁₀ alkenyl, C₃-C₁₀ alkynyl and (CR^{a}R^{b})ₙAr, wherein n is an integer ranging from 0 to 10, and wherein one or more carbons of said alkyl are optionally replaced by one or more diradicals selected from -O-, -S-, -S(O)-, -S(O)₂-, -NH-, -N(C₁₋C₆)alkyl- and -C(O)- and are optionally substituted by one or more substituents selected from the group T substituents;
R³ is selected from H, C(O)(C₁-C₁₈)alkyl, C(O)Ar, OC(O)(C₁-C₁₈)alkyl and OC(O)Ar, wherein the alkyl moieties of the foregoing R³ groups are optionally replaced by one or more diradicals selected from -O-, -S-, -S(O)-, -S(O)₂-, -NH-, -N(C₁-C₆)alkyl- and -C(O)- and are optionally substituted by one or more substituents selected from the group T substituents;
each of R⁴, R⁵ and R⁶ is independently selected from H, C₁-C₁₂ alkyl, C₅-C₁₀ aryl, and C₃-C₁₀ heterocyclic, wherein one or more carbons of said alkyl are optionally replaced by one or more diradicals selected from -O-, -S-, -S(O)-, -S(O)₂-, -NH-, -N(C₁-C₆) alkyl- and -C(O)- and said alkyl, aryl, and heterocyclic groups are optionally substituted by one or more substituents selected from the group T substituents;
R⁵ and R⁶ together with the nitrogen to which they are attached can form a 3- to 10-membered ring, in which one or more carbons are optionally replaced by one or more diradicals selected from -O-, -S-, -S(O)-, -S(O)₂-, -NH-, -N(C₁-C₆) alkyl- and -C(O)- and are optionally substituted by one or more substituents selected from the group T substituents;
R⁷ is selected from the group consisting of C₅-C₁₀ aryl, substituted C₅-C₁₀ aryl, 5- to 10- membered heteroaryl, substituted 5- to 10- membered heteroaryl and 3- to 10- membered heterocycloalkyl;
each of R⁸ and R⁹ is independently selected from the group consisting of C₁-C₁₂ alkenyl, C₁-C₁₂ alkynyl, C₅-C₁₀ aryl, C₃-C₈ cycloalkyl, 3- to 10- membered heterocycloalkyl and 5- to 10-membered heteroaryl, wherein said alkenyl, alkynyl, aryl, cycloalkyl, heterocycloalkyl and heteroaryl are optionally substituted by one or more substituents selected from the group T substituents;
R¹⁰ is -OCONR¹¹R¹²;
R¹¹ is H or C₁-C₆ alkyl;
R¹² is H, C₁-C₆ alkyl, -(CR¹³R¹⁴)ᵤ(C₃-C₁₀ cycloalkyl), -(CR¹³R¹⁴)ᵤ(C₅-C₁₀ aryl),-(CR¹³R¹⁴)ᵤ(3- to 10- membered heterocycloalkyl), (CR¹³R¹⁴)ᵤ(5- to 10-membered heteroaryl), wherein u is an integer from 0 to 10 and said aryl, cycloalkyl, heterocycloalkyl and heteroaryl in said R¹² group are optionally substituted by one or more substituents selected from the. group T substituents;
R¹³ and R¹⁴ are independently H or C₁-C₆ alkyl;
each of R^{a} and R^{b} is independently selected from H, halo and C₁-C₆ alkyl;
R^{a} and R^{b} together with the carbon to which they are attached can form a 3- to 10-membered cyclic or heterocyclic diradical, wherein one or more carbons of said diradical are optionally replaced by one or more diradicals selected from -O-, -S-, -S(O)-, -S(O)₂-, -NH-,-N(C₁-C₈)alkyl- and -C(O)- and are optionally substituted by one or more substituents selected from the group T substituents;
(CR^{a}R^{b})ₙ is alkylene, wherein n is an integer ranging from 0 to 10, uninterrupted or interrupted by one or more diradicals selected from -O-, -S-, -S(O)-, -S(O)₂-, -NH-, -N(C₁-C₆)alkyl- and -C(O)- and optionally substituted by one or more substituents selected from the group T substituents; or a compound of formula I wherein C-3 is substituted by (=O) in place of the sugar group shown.

2. The compound of claim 1 wherein R¹ is an alpha-branched C₃-C₈ alkyl, alkenyl, alkynyl, alkoxyalkyl or alkylthioalkyl group, any of which is optionally substituted by one or more hydroxyl groups; or a C₅-C₈ cycloalkylalkyl group wherein the alkyl group is an alpha-branched C₂-C₅ alkyl group, C₃-C₈ cycloalkyl or C₅-C₈ cycloalkenyl group, any of which may optionally be substituted by methyl or one or more hydroxyl or one or more C₁-C₄ alkyl groups or halo atoms; or a 3 to 6 membered oxygen or sulphur containing heterocyclic ring which may be saturated, or fully or partially unsaturated and which is optionally substituted by one or more C₁-C₄ alkyl groups or halo atoms.

3. The compound of claim 1 wherein Ar is selected from phenyl, 2-methoxyphenyl, 4-methoxyphenyl, quinolin-4-yl, 7-methoxy-quinolin-4-yl, 4-phenyl-imidazol-1-yl, pyridin-4-yl, pyridin-3-yl, pyridin-2-yl, 4-pyridinyl-1H-imidazol-1-yl, imidazo(45-b)pyridin-3-yl, 2-phenyl-thiazol-5-yl, 2-pyridin-3-yl-thiazol-4-yl and benzimidazol-1-yl; B is selected from NH, NMe and CH₂; and R² is (CH₂)ₙAr, wherein n is an integer ranging from 0 to 10.

4. The compound of claim 1 wherein R¹ is ethyl, R³ is H, R¹⁰ is -OC(O)NH₂, X¹ is -C(O)-, B is (CR^{aa}R^{bb})ₘ where m is 0 and R² is (CH₂)₄-(5- to 10- membered heteroaryl).

5. The compound of claim 1 wherein R¹ is ethyl, R³ is H, R¹⁰ is -OC(O)NH₂, X¹ is -C(O)-, and -B-R² is -NH(CH₂)₃-(5- to 10- membered heteroaryl).

6. The compound of claim 1 wherein B is (CR^{aa}R^{bb})ₘ where m is 0, R¹ ethyl, R² is H, R³ is H, X¹ is -C(O)-, and R¹⁰ is OC(O)NHR¹¹R¹²

7. A compound of claim 1 wherein B, R² and X¹ taken with their intervening atoms form an additional two rings having one of the following structures:

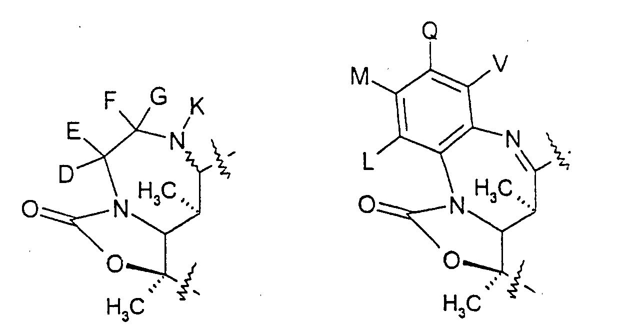

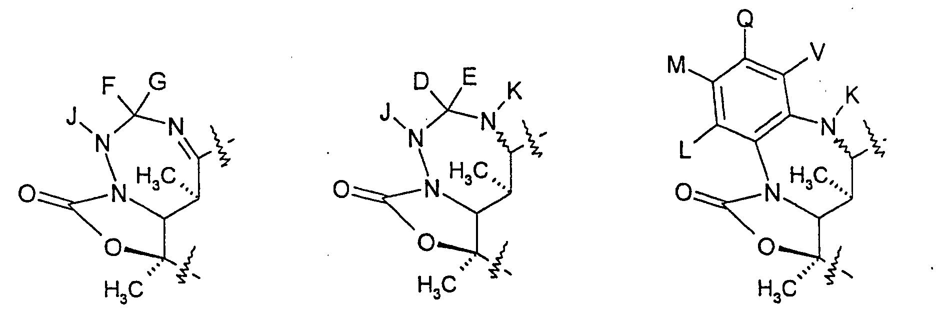

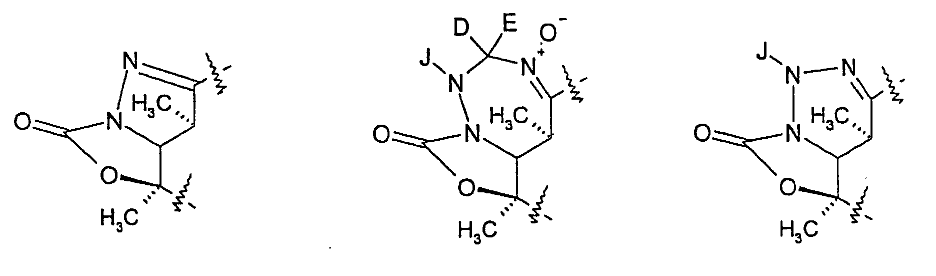

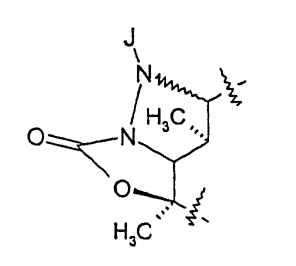

8. A pharmaceutical composition for the treatment of a bacterial infection or a protozoa infection in a mammal, fish, or bird which comprises a compound or a pharmaceutically acceptable salt, prodrug, tautomer, or solvate, thereof according to any one of claims 1 to 7, and a pharmaceutically acceptable carrier.

9. The use of a compound, salt, prodrug, tautomer or solvate according to any one of claims 1 to 7 in the manufacture of a medicament for the treatment of a bacterial infection or a protozoa infection.

10. A method of preparing a compound of claim 1 which comprises treating a compound of the formula 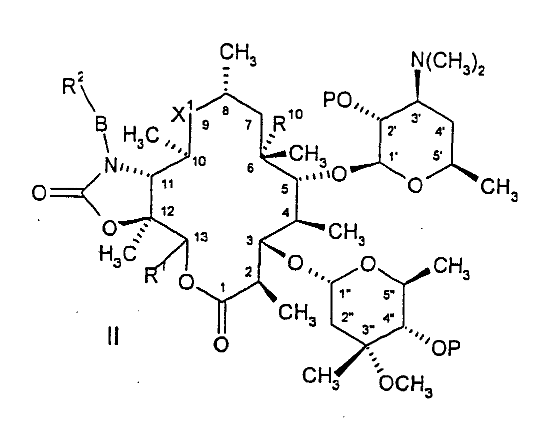 where P is a hydroxy protecting group , to remove the hydroxy protecting group.

11. A compound, salt, prodrug, tautomer or solvate according to any one of claims 1 to 7 for use in medicine.

12. A compound, salt, prodrug, tautomer or solvate according to any one of claims 1 to 7 for use in treating a bacterial infection or a protozoa infection.
